# EUROPÄISCHE PATENTSCHRIFT

(11) **EP 4 336 173 B1**
(45) Veröffentlichungstag und Bekanntmachung des Hinweises auf die Patenterteilung: **22.10.2025**
(21) Anmeldenummer: 23196637.5
(22) Anmeldetag: 11.09.2023
(51) Int. Cl.: G01N 21/71, G01N 33/205, F27B 1/28, F27B 3/02, F27B 3/28, F27D 21/00, F27D 27/00, G01N 21/84

(54) **VERFAHREN ZUR REGELUNG ODER STEUERUNG EINES SCHMELZOFENS UND SCHMELZOFEN, NÄMLICH SCHACHTOFEN, ZUM SCHMELZEN VON METALL**
METHOD OF CONTROLLING OR REGULATING A MELTING FURNACE AND MELTING FURNACE, NAMELY A SHAFT FURNACE, FOR MELTING METAL
PROCÉDÉ DE RÉGULATION OU DE COMMANDE D'UN FOUR DE FUSION ET FOUR DE FUSION, À SAVOIR FOUR À CUVE, POUR LA FUSION DE MÉTAL

(30) Priorität: 09.09.2022 EP 22194945
(43) Veröffentlichungstag der Anmeldung: 13.03.2024
(73) Patentinhaber: AMAG casting GmbH, 5282 Braunau am Inn-Ranshofen (AT)
(72) Erfinder: MÖNIUS, Martin, 84359 Simbach am Inn (DE); Faschang, Eduard, 4952 Weng (AT)
(74) Vertreter: Jell, Friedrich

(56) Entgegenhaltungen:
- EP-A1- 1 146 304
- EP-A1- 3 023 771
- WO-A1-2010/081807
- WO-A1-99/50466
- DE-A1- 102017 104 241
- GB-A- 2 154 315
- US-A1- 2011 222 057
- "Ullmann's Encyclopedia of Industrial Chemistry", 15 January 2003, WILEY-VCH, Weinheim, ISBN: 978-3-527-30673-2, article GRZELLA JÖRG ET AL: "Metallurgical Furnaces", pages: 693 - 736, XP055837395, DOI: 10.1002/14356007.b04_339

## Beschreibung

Die Erfindung betrifft einen Schmelzofen, nämlich Schachtofen, zum Schmelzen von Metall, mit mindestens einer Ofenkammer zur Aufnahme eines Metallschmelzbads, mit mindestens einer mit der Ofenkammer in Fluidverbindung stehenden Pumpeinrichtung, welche Pumpeinrichtung mindestens eine Metallschmelzpumpe zum Pumpen von Metallschmelze aus dem Metallschmelzbad aufweist, und mit einer Einrichtung zur laserinduzierten Plasmaspektroskopie (LIBS), die eine Laserquelle zum Senden eines gepulsten Laserstrahls auf die Metallschmelze zur Erzeugung eines Plasmas des Materials der Metallschmelze aufweist und die einen Spektralanalysator zum Analysieren der Strahlung des Plasmas zur Bestimmung der Zusammensetzung der Metallschmelze umfasst.

Aus der JPS6042644A ist ein Schmelzofen mit einer Einrichtung zur laserinduzierten Plasmaspektroskopie (LIBS als Abkürzung für Laser induced breakdown spectroscopy) bekannt, um anhand der Metallschmelze im Metallschmelzbad in der Ofenkammer metallurgische Messdaten erfassen zu können. Hierzu werden am Boden der Ofenkammer Gasblasen in das Metallschmelzbad eingebracht und für das LIBS-Verfahren verwendet. Nachteilig an dieser Maßnahme ist, dass sie sowohl konstruktiv vergleichsweise aufwendige Maßnahmen am Schmelzofen erfordert, als auch relativ anfällig gegenüber Verschmutzungen ist, was die Standfestigkeit der Einrichtung zur laserinduzierten Plasmaspektroskopie (LIBS) vermindern oder deren Messergebnisse verfälschen kann.

Das Dokument US2011/222057A1 offenbart die Überwachung der Zusammensetzung eines Schmelzbades zur Beschichtung eines Stahlbandes, wobei ein LIBS System an einer Öffnung einer Leitung des Schmelzbades angeordnet ist.

Die Erfindung hat sich daher die Aufgabe gestellt, einen Schmelzofen, nämlich einen Schachtofen, derart konstruktiv zu verändern, dass eine zuverlässige laserinduzierte Plasmaspektroskopie (LIBS) durchgeführt werden kann, um den Schachtofen damit verbessert steuern oder regeln zu können.

Die Erfindung löst die gestellte Aufgabe hinsichtlich des Schmelzofens durch die Merkmale des Anspruchs 1.

Indem die Pumpeinrichtung eine höher als die Metallschmelze und außerhalb der Ofenkammer angeordnete Zugangsöffnung aufweist, und die Einrichtung zur laserinduzierten Plasmaspektroskopie (LIBS) durch diese Zugangsöffnung auf die Metallschmelze wirkt, kann der Abgriff von Messdaten zur Metallschmelze in einen vergleichsweise zugänglichen Bereich des Schmelzofens verlagert werden. Zudem kann auch die Pumpeinrichtung der LIBS-Einrichtung eine repräsentative Probe der Metallschmelze in der Ofenkammer zur Verfügung stellen, was die Genauigkeit der Messung garantiert. Beispielsweise kann damit eine zuverlässige Abschätzung zu einer weiteren Entwicklung der Elementkonzentrationen möglich werden, was für eine verbesserte Steuerung/Regelung des Schmelzofens nutzbar ist. Erfindungsgemäß kann somit nicht nur die Konstruktion des Schmelzofens im Bereich der LIBS-Messdatenerfassung vereinfacht, sondern auch die Genauigkeit der LIBS-Messdatenerfassung erhöht werden.

Die Konstruktion kann weiter vereinfacht werden, wenn die Pumpeinrichtung eine Pumpentasche aufweist, wobei die Zugangsöffnung an der Pumpentasche, insbesondere an deren Deckel, vorgesehen ist.

Zudem ist vorstellbar, dass die Pumpeinrichtung einen Charge- und/oder einen Side-well aufweist, wobei die Zugangsöffnung am Charge- und/oder Side-well, insbesondere an dessen Deckel, vorgesehen ist. Denn auch hier ist handhabungsfreundlich eine standfeste Messdatenerfassung zu ermöglichen.

Vorzugsweise weist die Einrichtung zur laserinduzierten Plasmaspektroskopie (LIBS) eine Messlanze auf, die durch die Zugangsöffnung auf die Metallschmelze zu bewegbar ausgebildet ist, wodurch im Nahbereich der Metallschmelze eine zuverlässige Messdatenerfassung möglich werden kann. Dies kann die Qualität der LIBS-Messdatenerfassung weiter erhöhen.

Vorzugsweise läuft die Messlanze an ihrem freien Ende konusförmig zu, um damit die Angriffsfläche am Messkopf der Messlanze zu minimieren. Dies kann die Standfestigkeit der LIBS-Einrichtung noch weiter erhöhen.

Vorzugsweise weist die Wand der Messlanze eine Keramikverbindung auf oder besteht aus einer Keramikverbindung, um den vergleichsweise hohen Temperaturen bei der Messdatenerfassung standhalten zu können. Zudem kann damit der Messkopf nahe an die Metallschmelze zugefahren werden, was die LIBS-Messdatenerfassung in ihrer Genauigkeit weiter verbessern kann.

Die Metallschmelze kann von aufschwimmenden Verunreinigungen, beispielsweise Aluminiumkrätze, befreit werden, wenn die Messlanze an dem freien Ende mindestens eine Ausblasöffnung für ein durch die Messlanze geführtes Gas aufweist. Zudem ist die Messlanze mit dem durch sie geführten Gas zur Erhöhung ihrer Standfestigkeit auch zu kühlen.

Ist die Messlanze hohl, kann dies das Vorsehen von optischen Komponenten, die beispielsweise an die Laserquelle und/oder den Spektralanalysator angeschlossen sind, erleichtern.

Vorzugsweise steht die Pumpeinrichtung in Fluidverbindung mit ihrem Ein- und Auslass an der Ofenkammer zur Zirkulation oder Umwälzung der Metallschmelze des Metallschmelzbads in der Ofenkammer. Damit kann ein äußerst genauer Rückschluss auf die Metallschmelze im Metallschmelzbad der Ofenkammer erreicht werden. Die Messgenauigkeit ist damit weiter erhöhbar.

Vorstellbar ist weiter, dass die Pumpeinrichtung mit ihrem Einlass an der Ofenkammer und ihrem Auslass an der Schachtkammer des Schmelzofens zum Eintrag von Metallschmelze in die Schachtkammer in Fluidverbindung steht. Dadurch ist eine weiter erhöhte konstruktive Einfachheit gegeben.

Vorzugsweise ist die Pumpeinrichtung zur Umwälzung der Metallschmelze des Metallschmelzbads ausgebildet. Damit kann die Pumpeinrichtung beispielsweise eine Homogenisierung der Metallschmelze im Schmelzofen sicherstellen.

Vorstellbar ist, dass die Schachtkammer einen Schacht mit einer Säule aus aufzuschmelzendem Metall aufweist. Oben auf diese Säule im Schacht wird das aufzuschmelzende Metall aufgebracht.

Die Erfindung hat sich außerdem die Aufgabe gestellt, ein Verfahren zur Steuerung oder Regelung eines Schmelzofens zu verbessern, um ein Halbzeug mit vorgegebener Soll-Zusammensetzung exakt herstellen zu können.

Die Erfindung löst die gestellte Aufgabe zum Verfahren mit den Merkmalen des Anspruchs 12.

Indem über die Zugangsöffnung des Schmelzofens mit dem gepulsten Laserstrahl ein Plasma des Materials der Metallschmelze erzeugt wird und mit einem Spektralanalysator die Strahlung des Plasmas analysiert wird, kann eine zuverlässige und genaue Analyse durchgeführt werden. Wird diese Analyse zur Bestimmung der Ist-Zusammensetzung der Metallschmelze verwendet, ist es möglich durch Zugabe von aufzuschmelzendem Metall die Ist-Zusammensetzung auf eine Soll-Zusammensetzung der Metallschmelze anzupassen. Daraus eröffnet sich die Möglichkeit, Halbzeuge mit vorgegebener Soll-Zusammensetzung exakt herzustellen.

Die Analyse ist weiter verbesserbar, wenn die Messlanze durch die Zugangsöffnung zur Metallschmelze im Abstand zu dieser vorgeschoben wird, die Metallschmelze mit durch die Messlanze strömenden Gas beaufschlagt wird, und das Plasma des Materials der Metallschmelze mit dem durch die Messlanze verlaufenden und gepulsten Laserstrahl erzeugt wird. Auf diese Weise kann die Steuerung und/oder Regelung des Schmelzofens weiter verbessert werden.

Vorzugsweise wird vor und/oder nach der Bestimmung der Ist-Zusammensetzung der Metallschmelze die laserinduzierte Plasmaspektroskopie (LIBS) an einer Referenzprobe mit bekannter Zusammensetzung kalibriert, um die Steuerung und/oder Regelung des Schmelzofens weiter zu verbessern.

Das erfindungsgemäße Verfahren kann sich insbesondere dafür eignen, aus der dem Schmelzofens entnommenen Metallschmelze ein Halbzeug, insbesondere aus einer Aluminiumlegierung, herzustellen.

In den Figuren ist beispielsweise der Erfindungsgegenstand anhand einer Ausführungsvariante näher dargestellt. Es zeigen
- Fig. 1: eine schematische Ansicht des Schmelzofens,
- Fig. 2: eine Schnittansicht zu einer ersten Pumpeinrichtung des Schmelzofens nach Fig. 1 mit einer Einrichtung zur laserinduzierten Plasmaspektroskopie (LIBS) bei einer Messdatenaufnahme und
- Fig. 3: die Einrichtung zur laserinduzierten Plasmaspektroskopie (LIBS) nach Fig. 2 bei einer Messkalibrierung.

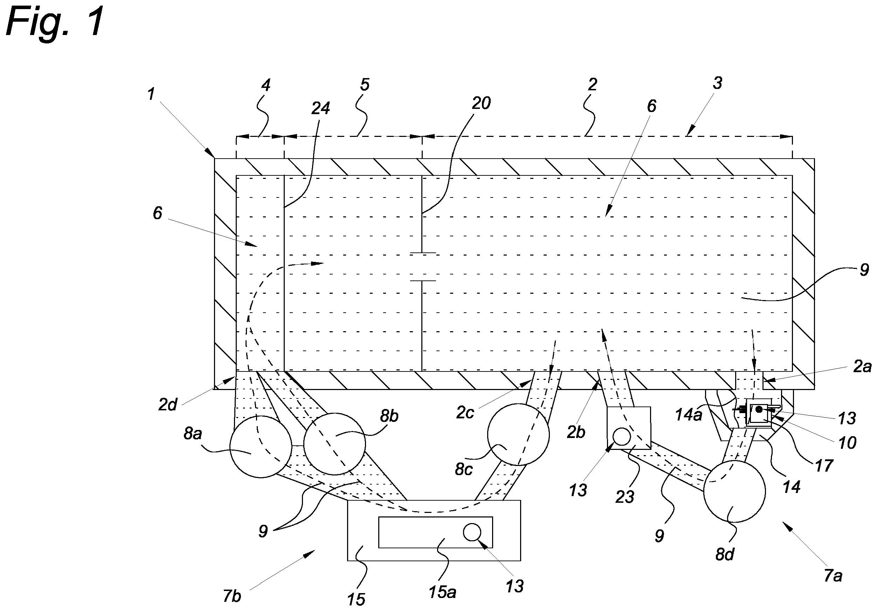

Nach Fig. 1 wird ein Schmelzofen 1, nämlich Schachtofen, zum Schmelzen, vorzugsweise Tauchschmelzen, von Metall, vorzugsweise Nichteisenmetallen, schematisch dargestellt. Vorzugsweise wird damit auch Schrott, insbesondere Aluminiumschrott, umgeschmolzen. Der Schmelzofen 1 wird mit einer nicht dargestellten Brenneranlage beheizt. Beim Schmelzofen 1 handelt es sich um einen Aluminium-Schachtschmelzofen.

Dieser mehrkammerige, nämlich dreikammerige, Schachtofen 1 weist eine Ofenkammer 2 als Hauptkammer 3 und eine Schachtkammer 4 zur Metallzufuhr sowie eine Nebenkammer 5 zwischen Schachkammer 4 und Ofenkammer 2 auf. In der Ofenkammer 2 befindet sich ein Metallschmelzbad 6, das über die Nebenkammer 5 in die Schachtkammer 4 reicht. Zwischen Ofenkammer 2 und Nebenkammer 5 ist eine Tauchwand 20 vorgesehen. Zwischen Schachtkammer 4 und Nebenkammer 5 ist eine Schachtwand 24 vorgesehen. Entsprechend der Ofenkonstruktion als Schachtofen wird das aufzuschmelzende Metall der Schachtkammer 4 zugeführt, und zwar oben auf eine Säule aus aufzuschmelzendem Metall im Schacht der Schachtkammer 4. Das aufzuschmelzende Metall sinkt im Schacht ab, erwärmt sich dabei und tritt dann in das Metallschmelzbad 6 ein.

Mit der Ofenkammer 2 stehen eine erste und eine zweite Pumpeinrichtung 7a und 7b in Fluidverbindung. Die zwei Pumpeinrichtungen 7a, 7b weisen Metallschmelzpumpen 8a bis 8d zum Pumpen der Metallschmelze 9 aus dem Metallschmelzbad 6 auf. Die Pumpeinrichtungen 7a, 7b dienen zur Umwälzung der Metallschmelze 9 des Metallschmelzbads 6. Diese Umwälzung dient beispielsweise zur Homogenisierung der Metallschmelze 9 des Metallschmelzbad 6.

In weiterer Folge wird auf die erste Pumpeinrichtung 7a der Fig. 1 näher eingegangen. Diese erste Pumpeinrichtung 7a fördert die Metallschmelze 9 wieder zurück in das Metallschmelzbad 6. Hierzu steht die erste Pumpeinrichtung 7a mit einem Ein- und einem Auslass 2a, 2b an der Ofenkammer 2 in Fluidverbindung. Damit wird die Metallschmelze 9 des Metallschmelzbads 6 in der Ofenkammer 2 in Strömungsrichtung (vgl. Fig. 1, Pfeilrichtung) umgewälzt.

Zudem weist der Schmelzofen 1 eine Einrichtung 10 zur laserinduzierten Plasmaspektroskopie (LIBS) auf. Diese LIBS-Einrichtung 10 ist mit einer Laserquelle 11 und einem Spektralanalysator 12 versehen. Dabei dient die Laserquelle 11 zum Senden eines gepulsten Laserstrahls 11a auf die Metallschmelze 9, um damit ein Plasma des Materials der Metallschmelze 9 zu erzeugen. Der Spektralanalysator 12 analysiert die Strahlung des erzeugten Plasmas und bestimmt daraus die Zusammensetzung der Metallschmelze 9.

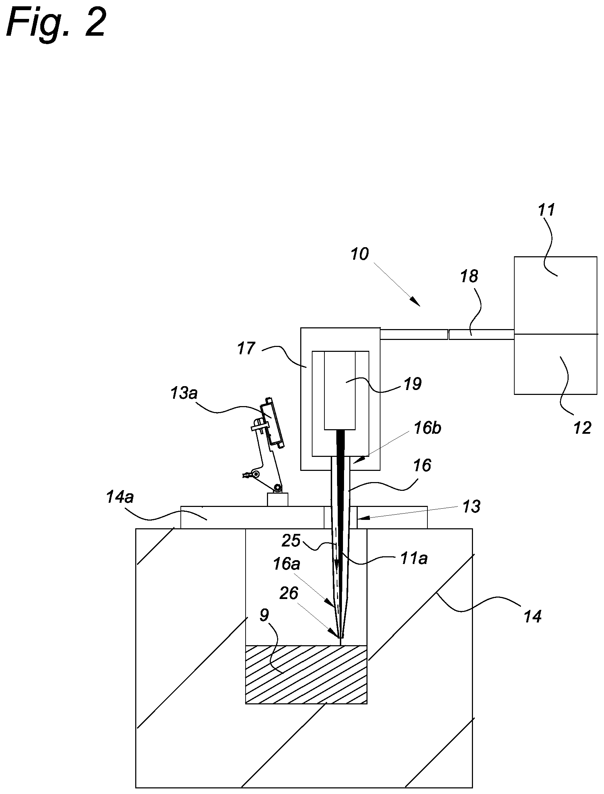

Erfindungsgemäß wird das LIBS-Verfahren nicht an der Metallschmelze 9 in der Ofenkammer 2, sondern außerhalb dieser Ofenkammer 2 vorgenommen. Hierzu weist die Pumpeinrichtung 7a, 7b eine höher als die Metallschmelze 9 (also über deren Pegel) und außerhalb der Ofenkammer 2 angeordnete Zugangsöffnung 13 auf. Vorzugsweise ist diese Zugangsöffnung 13 direkt über der Metallschmelze 9 angeordnet, wie in der Fig. 2 zu erkennen. Die LIBS-Einrichtung 10 wirkt nun durch die Zugangsöffnung 13 auf die Metallschmelze 9, um die Messung durchzuführen. Damit ist die LIBS-Einrichtung 10 von den widrigen Bedingungen in der Ofenkammer 2 geschützt angeordnet und kann die Messung genau und standfest durchführen. Auch ist die Metallschmelze 9 an den beiden Pumpeinrichtungen 7a und 7b durch die direkte Entnahme aus der Ofenkammer 2 eine vergleichsweise repräsentative Probe der Ofenkammer 2, was die Genauigkeit in der Bestimmung des Zustands der Schmelze in der Ofenkammer 2 erhöht.

Durch diesen optischen Zugang wird mittels des LIBS-Verfahrens die Legierungszusammensetzung ermittelt sowie in einem entsprechenden System gespeichert und verfügbar gemacht. Die Anzahl an Elementbestimmungen kann hierbei mehrmals pro Sekunde erfolgen. Diese Daten werden dann zum einen direkt ausgegeben, zum anderen mit den Daten des eingesetzten Materials verknüpft, sodass eine Aussage über die weitere Entwicklung der Elementkonzentrationen in der Metallschmelze 9 des Metallschmelzbads 6 ermöglicht wird. Dementsprechend kann beispielsweise durch ein weiter der Metallschmelze 9 zuzuführendes Material die Ist-Legierungszusammensetzung an eine geforderte Soll-Legierungszusammensetzung angepasst werden. Eine in der Zusammensetzung exakte Herstellung von Halbzeugen ist mit solch einer Regelung oder Steuerung des Schmelzofens 1 zu ermöglichen.

Wie in Fig. 1 und 2 zu erkennen, weist die erste Pumpeinrichtung 7a eine Pumpentasche 14 an der Metallschmelzpumpe 8d auf. Dort ist auch die Zugangsöffnung 13 vorgesehen, nämlich am abnehmbaren Deckel 14a der Pumpentasche 14. Die Zugangsöffnung 13 kann mit einem Verschluss 13a geöffnet und verschlossen werden.

Zudem befindet sich in Strömungsrichtung hinter der Metallschmelzpumpe 8d an der ersten Pumpeinrichtung 7a ein Charge-well 23, um beispielsweise einzuschmelzendes Metall dem Metallschmelzbad 6 der Ofenkammer 2 direkt zuführen zu können. Auf diesem Charge-well 23 ist ebenso eine Zugangsöffnung 13 vorgesehen sein, mit der ein Wirken der LIBS-Einrichtung 10 auf die Metallschmelze 9 möglich ist.

Damit die LIBS-Einrichtung 10 nahe an die Metallschmelze 9 zufahren kann, weist diese eine keramische Messlanze 16 mit einem daran angeschlossenen, in einem Hitzeschutzgehäuse montierten Messkopf 17 auf. Die Messlanze 16 verläuft am freien Ende 16a konisch. Die Messlanze 16 weist am anderen Ende 16b einen Messkopf 17 auf, der über einen Lichtleiter 18 mit der Laserquelle 11 und dem Spektralanalysator 12 verbunden ist - was in der Fig. 2 schematisch dargestellt ist. Im Messkopf 17 ist zudem einen Optik 19 beispielsweise zur Fokussierung des übertragenen Laserstrahls 11a und/oder zur Aufnahme des Plasmalichts vorgesehen.

Die Messlanze 16 ist durch die Zugangsöffnung 13 auf die Metallschmelze 9 vertikal zu bewegbar ausgebildet - wie in Fig. 2 zu angedeutet. Damit ist die LIBS-Einrichtung 10 in der Lage, den Abstand zur Metallschmelze 9 selbstständig einzustellen, beispielsweise durch eine Abstandsregelung oder -steuerung, um so eine permanente Fokussierung des Laserstrahls 11a sicherzustellen. Zudem kann Gas 25 über die zentrische Öffnung 26, durch die auch der Laserstrahls 11a austritt, am freien Ende 16a der Messlanze 16 auf die Metallschmelze 9 aufgeblasen werden, um aufschwimmende Verunreinigungen von der Messstelle wegzudrängen und damit zu entfernen. Auch eine Kühlung ist anhand des Gases möglich. Damit wird stets eine exakte Analyse gewährleistet.

Eine Anpassung an die verschiedenen Füllstände erfolgt beispielsweise durch die Nutzung von Verschub-Elementen, welche die LIBS-Einrichtung 10 anhand des gemessenen Ofenfüllstands positionieren.

Die Fokussierung über den kompletten Füllstandsbereich an Metallschmelze 9 in der Pumpeinrichtung 7, beispielsweise in der Pumpentasche 14, Charge-well 23 oder Side-well 15, kann erreicht werden, indem die gesamte LIBS-Einrichtung 10 auf einer vertikal und horizontal verfahrbaren Plattform angeordnet ist, welche in der Nähe des Schmelzofens 1 oder direkt am Schmelzofen 1 befestigt ist, was in den Figuren nicht dargestellt wurde.

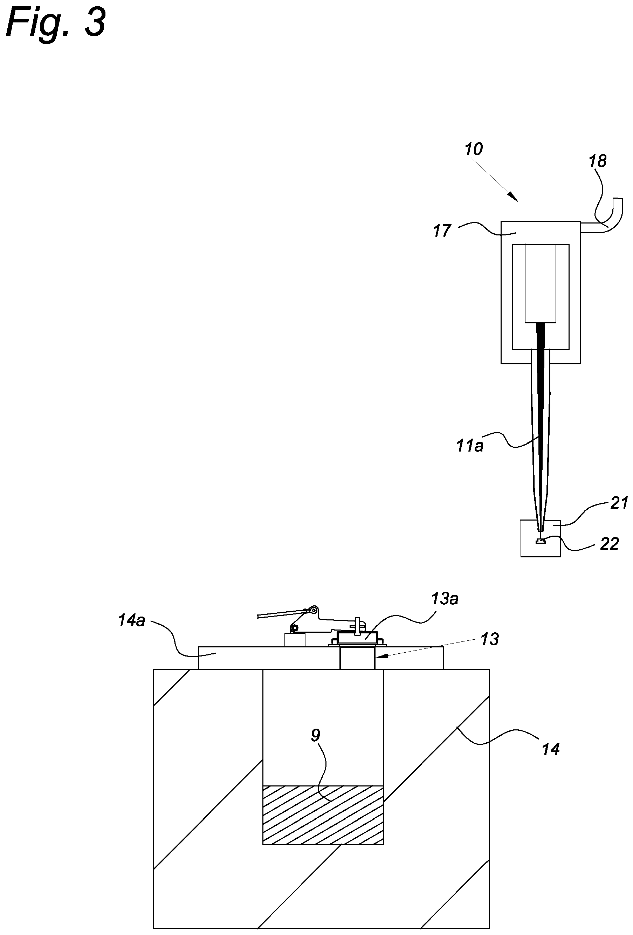

Für einen regelmäßigen Abgleich der LIBS-Einrichtung 10 ist eine Validierstation 21 mit einer in der Zusammensetzung bekannten Referenzprobe 22 vorgesehen - siehe hierzu Fig. 3. Durch Kalibrierung der LIBS-Einrichtung 10 an der bekannten Zusammensetzung der Referenzprobe 22 werden Zuverlässigkeit und Wiederholbarkeit der Messergebnisse der Metallschmelze 9 gewährleistet.

Alternativ ist vorstellbar, dass bei jener zweiten Pumpeinrichtung 7b gemessen wird, welche Metallschmelze 9 mithilfe von Metallschmelzpumpen 8a, 8b oder 8c von der Ofenkammer 2 in die Schachtkammer 4 des Schmelzofens pumpt und damit umgewälzt - wie in Fig. 1 zu erkennen.

Die zweite Pumpeinrichtung 7b kann hierbei auch ein Side-well 15, beispielsweise zwischen den Metallschmelzpumpen 8a, 8b und 8c, aufweisen. Der Side-well 15 kann beispielsweise verwendet werden, um oxidische Anhäufungen aus dem Schmelzprozess zu entfernen.

Eine alternative Zugangsöffnung 13 zur LIBS-Messdatenerfassung ist beispielsweise am Side-well 15, nämlich am Deckel 15a, vorgesehen.

## Patentansprüche

1. Schmelzofen, zum Schmelzen von Metall, mit mindestens einer Ofenkammer (2) zur Aufnahme eines Metallschmelzbads (6), mit mindestens einer mit der Ofenkammer (2) in Fluidverbindung stehenden Pumpeinrichtung (7a, 7b), welche Pumpeinrichtung (7a, 7b) mindestens eine Metallschmelzpumpe (8a, 8b, 8c, 8d) zum Pumpen von Metallschmelze (9) aus dem Metallschmelzbad (6) aufweist, und mit einer Einrichtung (10) zur laserinduzierten Plasmaspektroskopie, LIBS, die eine Laserquelle (11) zum Senden eines gepulsten Laserstrahls (11a) auf die Metallschmelze (9) zur Erzeugung eines Plasmas des Materials der Metallschmelze (9) aufweist und die einen Spektralanalysator (12) zum Analysieren der Strahlung des Plasmas zur Bestimmung der Zusammensetzung der Metallschmelze (9) umfasst, wobei die Pumpeinrichtung (7a, 7b) eine höher als die Metallschmelze (9) und außerhalb der Ofenkammer (2) angeordnete Zugangsöffnung (13) aufweist, und dass die Einrichtung (10) zur laserinduzierten Plasmaspektroskopie, LIBS, durch diese Zugangsöffnung (13) auf die Metallschmelze (9) wirkt,
**dadurch gekennzeichnet,**
**dass** der Schmelzofen ein Schachtofen mit einer Schachtkammer (4) zur Metallzufuhr ist.

2. Schmelzofen nach Anspruch 1, **dadurch gekennzeichnet, dass** die Pumpeinrichtung (7a) eine Pumpentasche (14) aufweist, wobei die Zugangsöffnung (13) an der Pumpentasche (14), insbesondere an deren Deckel (14a), vorgesehen ist.

3. Schmelzofen nach Anspruch 1 oder 2, **dadurch gekennzeichnet, dass** die Pumpeinrichtung (7a, 7b) einen Charge-well (23) und/oder einen Side-well (15) aufweist, wobei die Zugangsöffnung (13) am Charge-well (23) und/oder Side-well (15), insbesondere an dessen Deckel (13a oder 15a), vorgesehen ist.

4. Schmelzofen nach einem der Ansprüche 1 bis 3, **dadurch gekennzeichnet, dass** die Einrichtung (10) zur laserinduzierten Plasmaspektroskopie, LIBS, eine Messlanze (16) aufweist, die durch die Zugangsöffnung (13) auf die Metallschmelze (9) zu bewegbar ausgebildet ist.

5. Schmelzofen nach Anspruch 4, **dadurch gekennzeichnet, dass** die Messlanze (16) ihrem freien Ende (16a) konusförmig zuläuft und/oder dass die Wand der Messlanze (16) eine Keramikverbindung aufweist.

6. Schmelzofen nach Anspruch 4 oder 5, **dadurch gekennzeichnet, dass** die Messlanze (16) an ihrem freien Ende (16a) mindestens eine Ausblasöffnung (26) für ein durch die Messlanze (16) geführtes Gas (25) aufweist.

7. Schmelzofen nach einem der Ansprüche 4 bis 6, **dadurch gekennzeichnet, dass** die Messlanze (16) hohl ist.

8. Schmelzofen nach einem der Ansprüche 1 bis 7, **dadurch gekennzeichnet, dass** die Pumpeinrichtung (7a) mit ihrem Ein- und Auslass (2a, 2b) an der Ofenkammer (2) zur Zirkulation oder Umwälzung der Metallschmelze (9) des Metallschmelzbads (6) in der Ofenkammer (2) in Fluidverbindung steht.

9. Schmelzofen nach einem der Ansprüche 1 bis 7, **dadurch gekennzeichnet, dass** die Pumpeinrichtung (7b) mit ihrem Einlass (2c) an der Ofenkammer (2) und ihrem Auslass (2d) an der Schachtkammer (4) des Schmelzofens (1) zum Eintrag von Metallschmelze (9) in die Schachtkammer (4) in Fluidverbindung steht.

10. Schmelzofen nach einem der Ansprüche 1 bis 9, **dadurch gekennzeichnet, dass** die Pumpeinrichtung (7a, 7b) zur Umwälzung der Metallschmelze (9) des Metallschmelzbads (6) ausgebildet ist.

11. Schmelzofen nach einem der Ansprüche 1 bis 10, **dadurch gekennzeichnet, dass** die Schachtkammer (4) einen Schacht mit einer Säule aus aufzuschmelzendem Metall aufweist.

12. Verfahren zur Steuerung oder Regelung eines Schmelzofens (1) nach einem der Ansprüche 1 bis 11, bei dem über die Zugangsöffnung (13) des Schmelzofens (1) mit dem gepulsten Laserstrahl (11a) ein Plasma des Materials der Metallschmelze (9) erzeugt wird, mit einem Spektralanalysator (12) die Strahlung des Plasmas analysiert wird und diese Analyse zur Bestimmung der Ist-Zusammensetzung der Metallschmelze (9) verwendet wird, um durch Zugabe von aufzuschmelzendem Metall die Ist-Zusammensetzung auf eine Soll-Zusammensetzung der Metallschmelze (9) anzupassen.

13. Verfahren nach Anspruch 12, **dadurch gekennzeichnet, dass** die Messlanze (16) durch die Zugangsöffnung (13) zur Metallschmelze (9) im Abstand zu dieser vorgeschoben wird, die Metallschmelze (9) mit durch die Messlanze (16) strömenden Gas (25) beaufschlagt wird, und das Plasma des Materials der Metallschmelze (9) mit dem durch die Messlanze (16) verlaufenden und gepulsten Laserstrahl (11a) erzeugt wird.

14. Verfahren nach Anspruch 12 oder 13, **dadurch gekennzeichnet, dass** vor und/oder nach der Bestimmung der Ist-Zusammensetzung der Metallschmelze (9) die laserinduzierte Plasmaspektroskopie, LIBS, an einer Referenzprobe (22) mit bekannter Zusammensetzung kalibriert wird.

15. Verfahren nach Anspruch 12, 13 oder 14, **dadurch gekennzeichnet, dass** aus der dem Schmelzofens (1) entnommenen Metallschmelze (9) ein Halbzeug, insbesondere aus einer Aluminiumlegierung, hergestellt wird.

## Claims

1. Melting furnace for melting metal, having at least one furnace chamber (2) for receiving a molten metal bath (6), having at least one pump device (7a, 7b) in fluid connection with the furnace chamber (2), which pump device (7a, 7b) has at least one metal melt pump (8a, 8b, 8c, 8d) for pumping molten metal (9) from the molten metal bath (6), and having a device (10) for laser-induced plasma spectroscopy, LIBS, which has a laser source (11) for emitting a pulsed laser beam (11a) onto the molten metal (9) to generate a plasma of the material of the molten metal (9) and which comprises a spectral analyzer (12) for analyzing the radiation of the plasma to determine the composition of the molten metal (9), wherein the pump device (7a, 7b) has an access opening (13) arranged higher than the molten metal (9) and outside the furnace chamber (2), and that the device (10) for laser-induced plasma spectroscopy, LIBS, acts through this access opening (13) on the molten metal (9), **characterized in that** the melting furnace is a shaft furnace having a shaft chamber (4) for supplying metal.

2. Melting furnace according to claim 1, **characterized in that** the pump device (7a) has a pump pocket (14), wherein the access opening (13) is provided on the pump pocket (14), more particularly on its cover (14a).

3. Melting furnace according to claim 1 or 2, **characterized in that** the pump device (7a, 7b) has a charge well (23) and/or a side well (15), wherein the access opening (13) is provided on the charge well (23) and/or side well (15), more particularly on its cover (13a or 15a).

4. Melting furnace according to one of claims 1 to 3, **characterized in that** the device (10) for laser-induced plasma spectroscopy, LIBS, has a measuring lance (16) which is designed to be movable through the access opening (13) toward the molten metal (9).

5. Melting furnace according to claim 4, **characterized in that** the measuring lance (16) conically tapers toward its free end (16a) and/or **in that** the wall of the measuring lance (16) has a ceramic compound.

6. Melting furnace according to claim 4 or 5, **characterized in that** the measuring lance (16) has at least one blow-out opening (26) at its free end (16a) for a gas (25) conducted through the measuring lance (16).

7. Melting furnace according to one of claims 4 to 6, **characterized in that** the measuring lance (16) is hollow.

8. Melting furnace according to one of claims 1 to 7, **characterized in that** the pump device (7a) is in fluid connection with the furnace chamber (2) via its inlet and outlet (2a, 2b) for circulating or recirculating the molten metal (9) of the molten metal bath (6) in the furnace chamber (2).

9. Melting furnace according to one of claims 1 to 7, **characterized in that** the pump device (7b) with its inlet (2c) on the furnace chamber (2) and its outlet (2d) on the shaft chamber (4) of the melting furnace (1) is in fluid connection for introducing molten metal (9) into the shaft chamber (4).

10. Melting furnace according to one of claims 1 to 9, **characterized in that** the pump device (7a, 7b) is designed to circulate the molten metal (9) of the molten metal bath (6).

11. Melting furnace according to one of claims 1 to 10, **characterized in that** the shaft chamber (4) has a shaft with a column of metal to be melted.

12. Method for controlling or regulating a melting furnace (1) according to one of claims 1 to 11, in which a plasma of the material of the molten metal (9) is generated via the access opening (13) of the melting furnace (1) with the pulsed laser beam (11a), having a spectral analyzer (12) with which the radiation of the plasma is analyzed, and this analysis is used to determine the actual composition of the molten metal (9) in order to adjust the actual composition to a desired composition of the molten metal (9) by adding metal to be melted.

13. Method according to claim 12, **characterized in that** the measuring lance (16) is advanced through the access opening (13) to the molten metal (9) at a distance therefrom, the molten metal (9) is charged with gas (25) flowing through the measuring lance (16), and the plasma of the material of the molten metal (9) is generated by the pulsed laser beam (11a) passing through the measuring lance (16).

14. Method according to claim 12 or 13, **characterized in that** before and/or after determining the actual composition of the molten metal (9), laser-induced plasma spectroscopy, LIBS, is calibrated on a reference sample (22) with a known composition.

15. Method according to claim 12, 13 or 14, **characterized in that** a semi-finished product, more particularly made of an aluminum alloy, is produced from the molten metal (9) taken from the melting furnace (1).

## Revendications

1. Four de fusion destiné à la fusion de métal, avec au moins une chambre de four (2) destinée à recevoir un bain de métal en fusion (6), avec au moins un équipement de pompage (7a, 7b) en liaison fluidique avec la chambre de four (2), lequel équipement de pompage (7a, 7b) présente au moins une pompe de métal en fusion (8a,8b,8c,8d) destinée à pomper du métal en fusion (9) à partir du bain de métal en fusion (6), et avec un équipement (10) destiné à la spectroscopie plasma induite par laser LIBS qui présente une source laser (11) destinée à émettre un faisceau laser (11a) pulsé sur le métal en fusion (9) pour produire un plasma du matériau du métal en fusion (9) et qui comprend un analyseur de spectre (12) destiné à analyser le rayonnement du plasma afin de déterminer la composition du métal en fusion (9), l'équipement de pompage (7a, 7b) présentant une ouverture d'accès (13) disposée plus haut que le métal en fusion (9) et en dehors de la chambre de four (2), et l'équipement (10) destiné à la spectroscopie plasma induite par laser LIBS agit à travers cette ouverture d'accès (13) sur le métal en fusion (9), **caractérisé en ce que** le four de fusion est un four à cuve avec une chambre de cuve (4) destinée à amener le métal.

2. Four de fusion selon la revendication 1, **caractérisé en ce que** l'équipement de pompage (7a) présente une poche de pompe (14), l'ouverture d'accès (13) étant prévue sur la poche de pompe (14), en particulier sur le couvercle (14a) de celle-ci.

3. Four de fusion selon la revendication 1 ou 2, **caractérisé en ce que** l'équipement de pompage (7a, 7b) présente un charge-well (23) et/ou un side-well (15), l'ouverture d'accès (13) étant prévue sur le charge-well (23) et/ou le side-well (15), en particulier sur le couvercle (13a ou 15a) de celui-ci.

4. Four de fusion selon l'une des revendications 1 à 3, **caractérisé en ce que** l'équipement (10) destiné à la spectroscopie plasma induite par laser LIBS présente une lance de mesure (16) qui est constituée de façon mobile vers le métal en fusion (9) à travers l'ouverture d'accès (13).

5. Four de fusion selon la revendication 4, **caractérisé en ce que** la lance de mesure (16) se termine en forme de cône vers son extrémité libre (16a) et/ou **en ce que** la paroi de la lance de mesure (16) présente une composition céramique.

6. Four de fusion selon la revendication 4 ou 5, **caractérisé en ce que** la lance de mesure (16) présente à son extrémité libre (16a) au moins une ouverture de soufflage (26) pour un gaz (25) conduit à travers la lance de mesure (16).

7. Four de fusion selon l'une des revendications 4 à 6, **caractérisé en ce que** la lance de mesure (16) est creuse.

8. Four de fusion selon l'une des revendications 1 à 7, **caractérisé en ce que**, avec son entrée et sortie (2a, 2b) sur la chambre de four (2), l'équipement de pompage (7a) est en liaison fluidique pour la circulation ou la recirculation du métal en fusion (9) du bain de métal en fusion (6) dans la chambre de four (2).

9. Four de fusion selon l'une des revendications 1 à 7, **caractérisé en ce que**, avec son entrée (2c) sur la chambre de four (2) et sa sortie (2d) sur la chambre de cuve (4) du four de fusion (1), l'équipement de pompage (7b) est en liaison fluidique pour l'introduction du métal en fusion (9) dans la chambre de cuve (4).

10. Four de fusion selon l'une des revendications 1 à 9, **caractérisé en ce que** l'équipement de pompage (7a, 7b) est constitué pour la recirculation du métal en fusion (9) du bain de métal en fusion (6).

11. Four de fusion selon l'une des revendications 1 à 10, **caractérisé en ce que** la chambre de cuve (4) présente une cuve avec une colonne en métal à mettre en fusion.

12. Procédé de commande ou de régulation d'un four de fusion (1) selon l'une des revendications 1 à 11, dans lequel, via l'ouverture d'accès (13) du four de fusion (1), un plasma du matériau du métal en fusion (9) est produit avec le faisceau laser (11a) pulsé, le rayonnement du plasma est analysé avec un analyseur de spectre (12) et cette analyse est utilisée pour la détermination de la composition effective du métal en fusion (9) pour adapter, par l'addition de métal à mettre en fusion, la composition effective à une composition de consigne du métal en fusion (9).

13. Procédé selon la revendication 12, **caractérisé en ce que** la lance de mesure (16) est avancée à travers l'ouverture d'accès (13) vers le métal en fusion (9) à distance de celui-ci, le métal en fusion (9) est alimenté en gaz (25) s'écoulant à travers la lance de mesure (16), et le plasma du matériau du métal en fusion (9) est produit avec le faisceau laser (11a) pulsé et traversant la lance de mesure (16).

14. Procédé selon la revendication 12 ou 13, **caractérisé en ce que**, avant et/ou après la détermination de la composition effective du métal en fusion (9), la spectroscopie plasma induite par laser LIBS est calibrée sur une éprouvette de référence (22) de composition connue.

15. Procédé selon la revendication 12, 13 ou 14, **caractérisé en ce que**, à partir du métal en fusion (9) prélevé dans le four de fusion (1), il est fabriqué un produit semi-fini, en particulier en alliage d'aluminium est fabriqué.
